(19) Europäisches Patentamt
European Patent Office
Office européen des brevets

(11) **EP 4 578 442 A1**

(12) **EUROPEAN PATENT APPLICATION**

(43) Date of publication:
**02.07.2025 Bulletin 2025/27**

(21) Application number: **24185615.2**

(22) Date of filing: **01.07.2024**

(51) International Patent Classification (IPC):
*A61K 9/00* (2006.01)     *A61K 9/50* (2006.01)
*A61K 45/06* (2006.01)     *A61M 37/00* (2006.01)
*A61K 31/567* (2006.01)

(52) Cooperative Patent Classification (CPC):
(C-Sets available)
A61K 9/5031; A61K 9/0021; A61K 9/5036;
A61K 31/567; A61M 37/0015          (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **26.12.2023 KR 20230191160**

(71) Applicant: **Small'Lab Co., Ltd.**
**Daejeon 34-027 (KR)**

(72) Inventors:
• **Kim, Byeong Su**
**34011 Daejeon (KR)**

• **Joo, So Kyoung**
**13490 Seongnam-si (KR)**
• **Choi, Hyuck**
**Namyangju-si 12221 (KR)**
• **Park, Hyeon Jun**
**16460 Suwon-si (KR)**
• **Lee, Jeong Min**
**16914 Yongin-si (KR)**
• **Lee, Jeong Gyu**
**34049 Daejeon (KR)**
• **Park, Chun Woong**
**30063 Sejong-si (KR)**

(74) Representative: **Ter Meer Steinmeister & Partner Patentanwälte mbB**
**Nymphenburger Straße 4**
**80335 München (DE)**

(54) **MICROSPHERE COMPOSITION FOR CONTRACEPTIVE MICRONEEDLES AND CONTRACEPTIVE MICRONEEDLES COMPRISING THE SAME**

(57)     The present invention provides a microsphere composition for contraceptive microneedles and contraceptive microneedles comprising the same.

The microsphere composition for contraceptive microneedles according to the present invention encapsulates a drug stably, exhibits sustained-release of the drug for at least one week, and has a particle size (Dv90) suitable for use in microneedles.

The contraceptive microneedles according to the present invention offer convenient administration with once-weekly use, superior skin drug permeability allowing for short attachment times, and sustained-release of the drug for at least one week. The contraceptive microneedle patch of the present invention provides sustained-release of the drug for at least one week even with short attachment times, making it useful as a female contraceptive.

FIG. 9

ME-6                    MD-18

(52) Cooperative Patent Classification (CPC): (Cont.)

C-Sets
**A61K 31/567, A61K 2300/00**

## Description

### Technical Field

The present invention relates to a microsphere composition for contraceptive microneedles and contraceptive microneedles comprising the same, more specifically to a microsphere composition for contraceptive microneedles comprising sustained-release estrogen microspheres and/or sustained-release progesterone microspheres as effective ingredients, and to contraceptive microneedles comprising said composition.

### Background Art

Contraceptives are primarily used to prevent unwanted pregnancies and are also widely utilized for managing menstrual cramps, irregular menstrual cycles, polycystic ovary syndrome, menorrhagia, and premenstrual syndrome.

Contraceptives utilize the components of estrogen and/or progesterone to artificially regulate hormone levels to inhibit ovulation. Contraceptives have been developed up to the fourth generation based on the presence and type of progesterone, with each generation showing differences in side effects. First-generation contraceptives, which were high-dose estrogen-only preparations, had severe side effects such as thrombosis and abnormal bleeding, leading to their discontinuation. Second-generation contraceptives mainly included levonorgestrel as the primary progesterone component but had side effects like acne and hirsutism. Third-generation contraceptives, which included gestodene, desogestrel, and norgestimate as the primary progesterone components, mitigated the side effects of hirsutism and acne caused by second-generation contraceptives, with commercially available products such as Melian™, Myvlar™, and Mercilon™.

These contraceptives are used as oral contraceptives, requiring users to take one pill daily at the same time for 21 days followed by a 7-day break. Failing to adhere to this schedule can lower the concentration of the active ingredients below the necessary levels for effective contraception, meaning users must carefully plan and adhere to their dosage cycle (Korean Laid-Open Publication No. 10-2007-0087141).

As an alternative to the inconvenient oral contraceptives, transdermal delivery contraceptives are emerging. Transdermal drug delivery can avoid many unwanted side effects and is particularly advantageous for drugs with short biological half-lives, allowing reduced total daily dosage and fewer chances of overdose or underdose. Additionally, transdermal systems provide the benefit of quickly terminating drug administration by removing the drug delivery system from the skin.

One example of a transdermal delivery contraceptive is a polymer patch that includes an adhesive polymer matrix (Korean Registered Patent No. 10-0758757). However, polymer patches have inherent issues with low skin permeability of the active ingredients due to the skin barrier, resulting in insufficient drug delivery efficiency. For optimal efficacy of contraceptives, the hormones, as the effective ingredients, must be released in sustained manner. However, for sustained release, a polymer patch must be adhered to the skin for extended periods each day, which can cause skin irritation and discomfort, particularly during activities like showering in the summer.

Therefore, there is a need to develop a transdermal contraceptive delivery system that offers convenient administration, superior skin drug permeability, and sustained drug release of the effective ingredients.

### Disclosure of the Invention

### Technical Problem to be Solved

As a result of continuous research to meet the demands of conventional techniques, the inventors have developed sustained-release estrogen microspheres and sustained-release progesterone microspheres for contraceptive microneedles. By incorporating these microspheres as active ingredients, they have successfully manufactured contraceptive microneedles that offer convenience in administration, excellent skin drug permeability, and sustained drug release, thus completing the present invention.

Therefore, the objective of the present invention is to provide a microsphere composition for contraceptive microneedles that offers convenient in administration, superior skin drug permeability, and sustained drug release of the effective ingredients.

Another objective of the present invention is to provide contraceptive microneedles comprising the said microsphere composition, offering convenient in administration, superior skin drug permeability, and sustained drug release of the effective ingredients.

Another objective of the present invention is to provide a contraceptive microneedle patch comprising the said microneedles.

## Solution to the Problem

To achieve the above objectives, the present invention provides a microsphere composition for contraceptive microneedles offering convenient administration, superior skin drug permeability, and sustained drug release of the effective ingredients.

In the present invention, the drug refers to estrogen and progesterone.

The microsphere composition for contraceptive microneedles comprises one selected from the group consisting of sustained-release estrogen microspheres, sustained-release progesterone microspheres, and mixtures thereof as effective ingredients.

In the present invention, "sustained-release" means controlled release of the drug encapsulated in the microspheres, preferably continuous release of 70-80% by weight of the drug within 120 hours.

In the present invention, sustained-release estrogen microspheres and sustained-release progesterone microspheres can be characterized by the release of estrogen and progesterone encapsulated in them lasting for at least one week, preferably for up to one week, within the body. These sustained-release estrogen microspheres and sustained-release progesterone microspheres are further characterized by continuously releasing 70-80% by weight of estrogen and progesterone encapsulated in them within 120 hours after entering the application environment (for example, after administration to the human body).

In the present invention, estrogen refers to ethinylestradiol (EE).

In the present invention, progesterone refers to one selected from the group consisting of desogestrel (DSG), gestodene, dienogest, levonorgestrel, norgestimate, norethisterone, dro-spirenone, trimegestone, and dydrogesterone.

In the present invention, sustained-release estrogen microspheres may comprise 400 to 600 parts by weight of a biodegradable polymer per 100 parts by weight of estrogen. Preferably, sustained-release estrogen microspheres comprise 400 parts by weight of a biodegradable polymer per 100 parts by weight of estrogen. When the estrogen content comprised in the microspheres falls within the aforementioned range, sustained release for at least one week is achievable.

In the present invention, a biodegradable polymer refers to a polymer that, when administered in vivo, degrades within the body without causing high cytotoxicity or inflammatory responses.

In the present invention, the particle size (Dv90) of the sustained-release estrogen microspheres is 20 $\mu$m or less, preferably 15 $\mu$m or less. If the particle size exceeds this range, the microspheres becomes too large to be included in microneedles.

In the present invention, the biodegradable polymers that can be used for sustained-release estrogen microspheres may be selected from the group consisting of poly(lactide-co-glycolide) (PLGA), polylactide (PLA), polyglycolide (PGA), poly(lactide-co-glycolide) glucose, and mixtures thereof. The most preferred is poly(lactide-co-glycolide) (PLGA).

In the present invention, sustained-release estrogen microspheres can be produced by preparing an O/W (oil-in-water) emulsion containing a biodegradable polymer, estrogen, and a solvent, and then aggregating this emulsion into microspheres using a single solvent evaporation method. Specifically, an oil phase containing estrogen and a solvent is prepared and dispersed into an aqueous phase containing a surfactant to produce an O/W emulsion.

The solvent can be selected from the group consisting of dichloromethane (DCM), chloroform, acetonitrile, dimethyl sulfoxide, dimethylformamide, ethyl acetate, and mixtures thereof, with dichloromethane (DCM) being the most preferred.

The surfactant can be selected from the group consisting of polyvinyl alcohol (PVA), polysorbate 20, polysorbate 60, polysorbate 80, and mixtures thereof, with polyvinyl alcohol (PVA) being the most preferred.

In the present invention, it is preferable that stirring for the preparation of the O/W emulsion is conducted at a speed of 1600 rpm or higher for at least 12 hours. If the stirring is performed at a speed lower than 1600 rpm, the particle size (Dv 90) of the microspheres becomes too large, making them unsuitable for inclusion in microneedles.

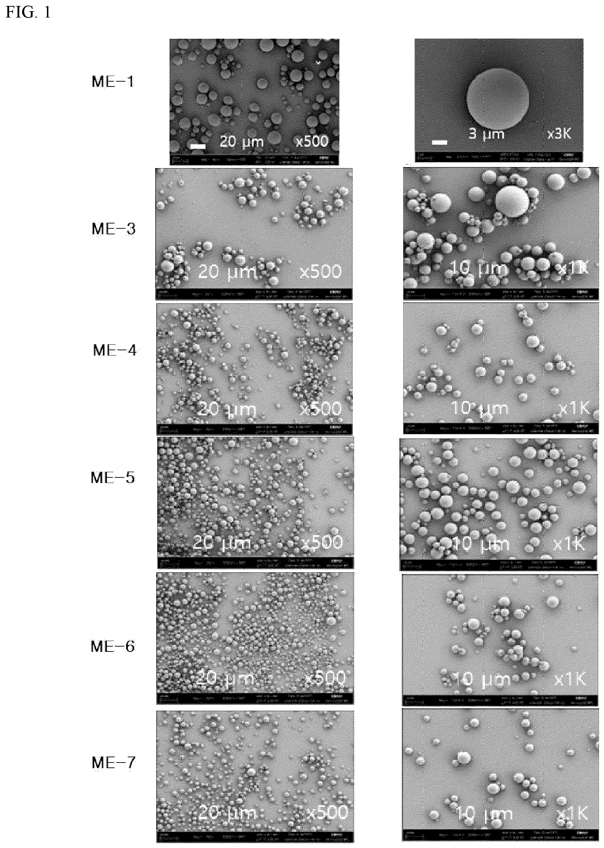

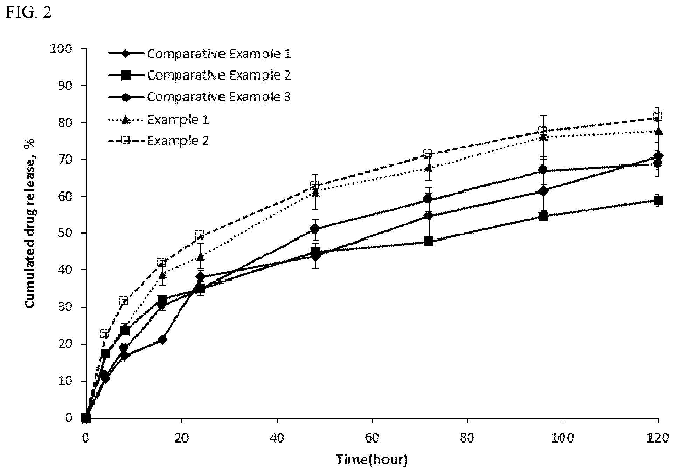

The sustained-release estrogen microspheres according to the present invention exhibit sustained release of estrogen for at least one week and have an average particle size suitable for inclusion in microneedles (FIG. 1, FIG. 2).

In the present invention, sustained-release progesterone microspheres may comprise 700 to 900 parts by weight of a biodegradable polymer per 100 parts by weight of progesterone. Most preferably, the sustained-release progesterone microspheres comprise 700 parts by weight of a biodegradable polymer per 100 parts by weight of progesterone.

The sustained-release progesterone microspheres according to the present invention may further comprise hydroxypropyl-beta-cyclodextrin (HP$\beta$CD) for stabilizing progesterone. In the sustained-release progesterone microspheres, hydroxypropyl-beta-cyclodextrin can be included at a molar ratio of progesterone to hydroxypropyl-beta-cyclodextrin of 1:2 to 1:3.

When the contents of progesterone and hydroxypropyl-beta-cyclodextrin in the sustained-release progesterone microspheres are within the above range, sustained release of progesterone for at least one week is possible.

The particle size (Dv90) of sustained-release progesterone microspheres according to the present invention is 20 $\mu$m or less, preferably 18 $\mu$m or less. If the particle size exceeds this range, the microspheres become unsuitable for inclusion in microneedles.

In the present invention, biodegradable polymers available for sustained-release progesterone microspheres can be selected from the group consisting of poly(lactide-co-glycolide) (PLGA) with a glycolic:lactic ratio of 1:2 to 4, poly(lactide-co-glycolide) glucose, and mixtures thereof. Poly(lactide-co-glycolide) (PLGA) with a glycolic:lactic ratio of 1:3 is the most preferred.

The sustained-release progesterone microspheres according to the present invention can be prepared using a W/O/W (water/oil/water) double solvent evaporation method. Specifically, an aqueous phase containing hydroxypropyl-beta-cyclodextrin dissolved in water is mixed with an oil phase containing a biodegradable polymer, progesterone, and a solvent. This mixture is then dispersed into an aqueous phase containing a surfactant to produce a W/O/W emulsion to aggregate into microspheres.

The solvent can be selected from the group consisting of dichloromethane (DCM), chloroform, acetonitrile, dimethyl sulfoxide, dimethylformamide, ethyl acetate, and mixtures thereof, with dichloromethane (DCM) being the most preferred.

During the dispersion, stirring at a speed of 1600 rpm or higher for at least 12 hours is preferred. If the stirring speed is lower than 1600 rpm, the average particle size of the microspheres becomes too large for inclusion in microneedles.

The surfactant can be selected from the group consisting of polyvinyl alcohol (PVA), polysorbate 20, polysorbate 60, polysorbate 80, and mixtures thereof, with polyvinyl alcohol (PVA) being the most preferred.

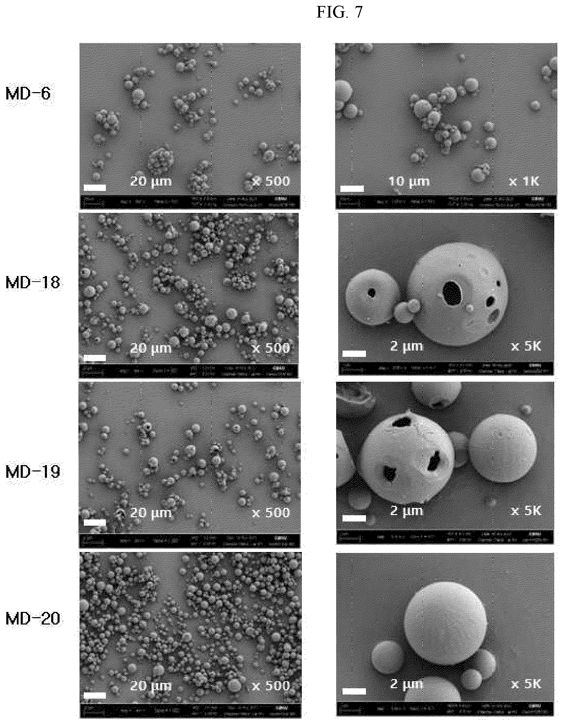

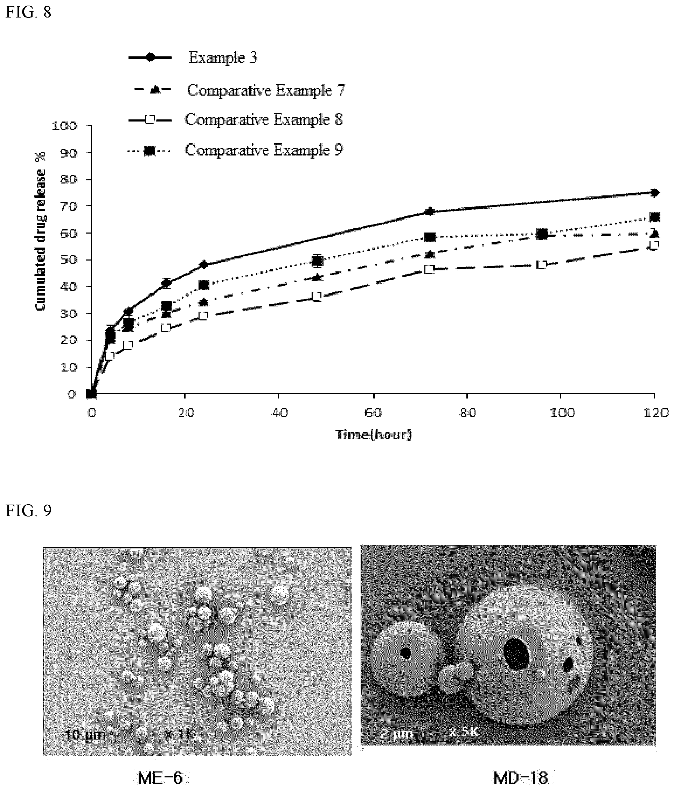

The sustained-release progesterone microspheres according to the present invention have a suitable particle size (Dv90) for inclusion in microneedles and have pores on their surface, providing sustained release of progesterone for at least one week (FIG. 7, FIG. 8).

Another objective of the present invention is to provide contraceptive microneedles comprising said microsphere composition.

The contraceptive microneedles in the present invention can be produced by mixing said microsphere composition with a soluble material, a stabilizer, and a solvent to prepare a solution, and then filling the solution in a mold to shape into microneedles.

In the present invention, the soluble material is biodegradable in vivo, forming the structure of the microneedles. Therefore, the microneedles according to the present invention are soluble in body fluids after skin insertion. The soluble material can be selected from the group consisting of hyaluronic acid (HA) or its salts, alginic acid (AA) or its salts, chitosan, collagen, gelatin, chondroitin, dextran, fibrin, agarose, pullulan, cellulose, polyvinylpyrrolidone (PVP), polyethylene glycol (PEG), polyvinyl alcohol (PVA), vinylpyrrolidone-vinyl acetate copolymer, hydroxypropyl cellulose, hydroxyethyl cellulose, hydroxypropylmethyl cellulose, sodium carboxymethyl cellulose (CMC), and mixtures thereof, with hyaluronic acid or its salts being the most preferred.

During the preparation of the microneedles according to the present invention, the soluble material can be included at 20-30% by weight of the total solution (100% by weight).

The stabilizer helps enhance the stability during the preparation of the microneedles and can be one or more selected from the group consisting of xylitol, trehalose, lactose, sucrose, polyhydric alcohols, cyclodextrins, dextrins, starch, glucose, maltose, lactulose, turanose, melibiose, melezitose, sorbitol, and mannitol.

During the preparation of the microneedles according to the present invention, the stabilizer can be included at 3-10% by weight of the total solution.

The microsphere composition of the present invention can be included at 1-10% by weight of the total solution during the preparation of the microneedles according to the present invention.

In the present invention, the solvent is water, preferably deionized water (D.W) or potassium phosphate buffer (PPB).

In the present invention, a pH adjuster as needed, with any pH adjuster commonly used in the preparation of microneedles, such as NaOH, may further added during the preparation of the microneedles, if needed.

In the present invention, plasticizers, surfactants, preservatives, etc., may further added during the preparation of the microneedles, if needed.

The microneedles of the present invention can include needle parts protruding in one direction and a matrix layer supporting the needle part. The needle part has a shape suitable for skin penetration. The shape of the needle parts of the microneedles of the present invention can be conical, pyramidal, spherical, truncated, wedge-shaped, blade-shaped, etc., and should commonly be a shape that can penetrate the skin. The length of the needle parts is 500-1000 $\mu$m, preferably 750 $\mu$m. The matrix layer has a thickness of 0.1-1 mm, preferably 0.1-0.3 mm.

The needle part of the microneedles of the present invention may be prepared to be separable from the matrix layer when inserted into the skin if necessary. The microneedles of the present invention can also have a matrix layer prepared from a different material, if necessary. Therefore, the microneedles of the present invention may have the microspheres evenly distributed in both the needle part and the matrix layer or only in the needle part.

The microneedles of the present invention are soluble microneedles that, upon biodegradation within the body, release sustained-release estrogen microspheres and/or sustained-release progesterone microspheres.

The microneedles of the present invention may include only sustained-release estrogen microspheres, only sustained-release progesterone microspheres, or both sustained-release estrogen microspheres and sustained-release progesterone microspheres.

The microneedles of the present invention offer convenient dosing with once-weekly administration, superior skin drug permeability by creating small holes in the skin, and sustained drug release for at least one week by using the sustained-release microspheres of the present invention.

Another objective of the present invention is to provide a contraceptive microneedle patch comprising said microneedles.

The contraceptive microneedle patch of the present invention has an adhesive layer laminated on one side of a matrix layer, allowing the microneedle patch to be attached to the skin for use. The microneedle patch can also include a protective film on the adhesive layer.

The contraceptive microneedle patch of the present invention offers sustained drug release for at least one week with short attachment times of less than 3 hours, making it useful as a female contraceptive.

**Advantageous Effects**

The microsphere composition for contraceptive microneedles of the present invention encapsulates the drug(s) stably, shows sustained drug release for at least one week, and has a suitable particle size (Dv90) for inclusion in microneedles.

The contraceptive microneedles of the present invention offer convenient dosing with once-weekly administration, superior skin drug permeability, and sustained drug release for at least one week with short attachment times.

The contraceptive microneedle patch of the present invention offers sustained drug release for at least one week with short attachment times, making it useful as a female contraceptive.

**Brief Description of the Drawings**

FIG. 1 shows scanning electron microscope (SEM) images of ethinylestradiol microspheres prepared in Preparation Example 1.

FIG. 2 is a graph showing the dissolution test results of ethinylestradiol microspheres prepared in Preparation Example 1.

FIG. 3 shows SEM images of desogestrel microspheres prepared in Preparation Example 2.

FIG. 4 is a graph showing the dissolution test results of desogestrel microspheres prepared in Preparation Example 2.

FIG. 5 is a graph showing the stability analysis results of desogestrel with different stabilizers.

FIG. 6 is a graph showing the stability analysis results with different molar ratios of desogestrel to stabilizers in the microspheres.

FIG. 7 shows SEM images of desogestrel microspheres prepared in Preparation Example 4.

FIG. 8 is a graph showing the dissolution test results of desogestrel microspheres prepared in Preparation Example 4.

FIG. 9 shows SEM images of the ethinylestradiol microspheres of Example 2 and the desogestrel microspheres of Example 3.

FIG. 10 shows images of the microneedles and their needle parts of Examples 4-6.

**Detailed Description of the Invention**

The present invention will now be described in more detail with reference to specific embodiments to aid understanding of the invention. However, these embodiments are merely examples for better understanding of the present invention and are not intended to limit the scope of the present invention.

**Preparation Example 1: Sustained-Release Ethinylestradiol Microspheres**

<Microsphere preparation>

Ethinylestradiol (EE) and PLGA 503H (Evonik Ltd. Germany) were added to 10 ml of dichloromethane (DCM) in the amounts shown in Table 1, mixed, and dissolved by sonication for 30 minutes to prepare an oil phase. The oil phase was dropped into 100 ml of polyvinyl alcohol solution (5% PVA in D.W) while stirring at the stirring speeds shown in Table 1, using a syringe pump at a rate of 2 ml/min to allow DCM to evaporate, for 12 hours, thus forming microspheres. The produced microspheres were precipitated using a centrifuge (2000 rpm for 10 minutes). The precipitated microspheres were filtered through a 0.2 μm PVDF membrane filter, washed twice with D.W, and dried at room temperature for 12 hours to obtain the microspheres.

**Table 1**

| Component | Comparative example 1 | | Comparative example 2 | | Comparative example 3 | | Example 1 | | Example 2 | | Comparative example 4 | |
|---|---|---|---|---|---|---|---|---|---|---|---|---|
| | ME-1 (1:10) | | ME-3 (1:10) | | ME-4 (1:8) | | ME-5 (1:6) | | ME-6 (1:4) | | ME-7 (1:2) | |
| | mg | % | mg | % | mg | % | mg | % | mg | % | mg | % |
| PLGA | 600 | 90.9 | 600 | 90.9 | 600 | 88.9 | 600 | 85.7 | 600 | 80.0 | 600 | 66.7 |
| EE | 60 | 9.1 | 60 | 9.1 | 75 | 11.1 | 100 | 14.3 | 150 | 20.0 | 300 | 33.3 |
| DCM (ml) | 10 | - | 10 | - | 10 | - | 10 | - | 10 | - | 10 | - |
| Toltal (Solute) | 660 | 100.0 | 660 | 100.0 | 675 | 100.0 | 700 | 100.0 | 700 | 100.0 | 700 | 100.0 |
| Rpm | 800 | | 1600 | | 1600 | | 1600 | | 1600 | | 1600 | |

<Yield, Loading, and Encapsulation Efficiency of Microspheres>

Using HPLC equipment (Agilent 1200 HPLC system), the drug encapsulated in the produced microspheres was analyzed, and the yield, loading, and encapsulation efficiency of the microspheres were calculated using the below formulas and shown in Table 2:

Yield: Actual yield / Theoretical yield $\times$ 100 (%)
Loading: Amount of loaded drug / Weight of microspheres $\times$ 100 (%)
Encapsulation efficiency: Actual drug loading / Theoretical drug loading $\times$ 100 (%)

**Table 2**

| | Comparative example 1 | Comparative example 2 | Comparative example 3 | Example 1 | Example 2 | Comparative example 4 |
|---|---|---|---|---|---|---|
| | ME-1 | ME-3 | ME-4 | ME-5 | ME-6 | ME-7 |
| Drug | EE | EE | EE | EE | EE | EE |
| Yield (%) | 59.4 | 75.5 | 78.9 | 76.4 | 68.9 | 77.2 |
| Loading (%) | 2.68±0.03 | 2.99±0.15 | 3.75±0.31 | 4.57±0.31 | 5.39±0.05 | 3.38±0.19 |
| Encapsulation efficiency(%) | 29.45±0.42 | 32.85±1.7 | 33.76±2.75 | 31.99±2.13 | 26.97±0.28 | 10.13±0.58 |

Comparative Example 4 showed low encapsulation efficiency (approximately 10.13%), making it unsuitable. It was confirmed that a higher amount of ethinylestradiol (EE) reduces encapsulation efficiency.

<Particle Shape and Size of Microspheres>

Using SEM equipment (Ultra Plus Carl Zeiss), the shapes of the particles of the microsphere were observed, and the results are shown in FIG. 1. All microspheres exhibited a spherical shape.
Additionally, particle sizes (Dv10, Dv50, Dv90) of the microspheres were measured using a Mastersizer 3000E equipment (wet measurement), and the results are shown in Table 3.

**Table 3**

| | | Dv10 (μm) | Dv50 (μm) | Dv90 (μm) | Span |
|---|---|---|---|---|---|
| Comparative example 1 | ME-1 | 8.17±0.01 | 16.9±0.28 | 36.57±2.62 | 1.67±0.12 |

(continued)

| | | Dv10 (μm) | Dv50 (μm) | Dv90 (μm) | Span |
|---|---|---|---|---|---|
| Comparative example 2 | ME-3 | 3.48±0.01 | 6.45±0.06 | 9.95±0.10 | 1.00±0.01 |
| Comparative example 3 | ME-4 | 1.50±0.01 | 4.88±0.02 | 9.77±0.11 | 1.69±0.01 |
| Example 1 | ME-5 | 1.98±0.01 | 6.25±0.05 | 12.07±0.31 | 1.60±0.03 |
| Example 2 | ME-6 | 1.61±0.01 | 4.88±0.07 | 10.50±0.46 | 1.82±0.06 |
| Comparative example 4 | ME-7 | 1.55±0.01 | 5.36±0.02 | 11.97±0.59 | 1.94±0.10 |

Comparative Example 1 showed a particle size (Dv90) of 36.57±2.62 μm, making it unsuitable for use in microneedles. It was confirmed that stirring at 1600 rpm or higher during microsphere preparation results in a particle size (Dv90) of around 10 μm, which is suitable for incorporation into microneedles.

<Dissolution Test - Sustained Release Test>

The sustained-release characteristics were analyzed by performing a dissolution test on the microspheres prepared as described above.

In a shaking bath (37°C, 50 rpm), 40 ml of dissolution solution (0.5% Tween 80 in PBS pH 7.4 1X) was added to a 50 ml conical tube. Microspheres were weighed to contain 1000 μg of ethinylestradiol (EE) and added to the dissolution solution for the dissolution evaluation.

Before sampling, the microspheres were precipitated using a centrifuge (1500 rpm, 5 min). The supernatant (10 ml) was sampled and analyzed by HPLC, and 10 ml of dissolution solution was replenished. Sampling times were set at 4, 8, 16, 24, 48, 72, 96, and 120 hours.

The dissolution time and dissolution rate results (up to less than 60% dissolution) were used to calculate kinetics according to the following equations:

Zero order: $Q_t = Q_0 + K_0 \times t$

First order: $\log C = \log C_0 - \dfrac{K_1}{2.303}$

Hixon-crowell: $W_0^{1/3} - W_t^{1/3} = K_{HC} \times t$

Higuchi: $Q = K_H \times t^{1/2}$

Korsmeyer-peppas:

$$\frac{M_t}{M_\infty} = K_{KP} \times t^n$$

The test results are shown in FIG. 2 and Tables 4 and 5.

**Table 4**

| Time (hr) | Comparative example 1 | Comparative example 2 | Comparative example 3 | Example 1 | Example 2 |
|---|---|---|---|---|---|
| | ME-1 | ME-3 | ME-4 | ME-5 | ME-6 |
| 0 | 0 | 0 | 0 | 0 | 0 |
| 4 | 10.7±0.35 | 17.3±0.57 | 11.5±0.96 | 17.2±0.60 | 22.6±0.53 |
| 8 | 16.8±0.20 | 23.7±0.76 | 18.6±1.31 | 24.5±1.08 | 31.4±0.65 |

(continued)

| Time (hr) | Comparative example 1 ME-1 | Comparative example 2 ME-3 | Comparative example 3 ME-4 | Example 1 ME-5 | Example 2 ME-6 |
|---|---|---|---|---|---|
| 16 | 21.2±0.56 | 32.1±0.97 | 30.4±1.49 | 38.7±2.81 | 41.8±0.79 |
| 24 | 38.1±1.75 | 35.0±0.22 | 35.0±1.82 | 43.8±3.44 | 49.2±0.62 |
| 48 | 43.7±3.51 | 45.0±0.75 | 50.9±2.78 | 61.2±4.64 | 62.6±0.54 |
| 72 | 54.7±6.15 | 47.8±0.23 | 59.1±3.25 | 67.8±3.58 | 71.2±0.44 |
| 96 | 61.5±5.20 | 54.6±1.29 | 66.9±3.65 | 76.1±5.88 | 77.6±1.10 |
| 120 | 70.8±3.69 | 59.0±1.59 | 68.8±3.54 | 77.7±6.24 | 81.4±1.02 |

**Table 5**

| | | Comparative example 2 ME-3 | Comparative example 3 ME-4 | Example 1 ME-5 | Example 2 ME-6 |
|---|---|---|---|---|---|
| | Kinetics | EE | EE | EE | EE |
| Zero order | $r^2$ | 0.9114 | 0.9071 | 0.8857 | 0.9018 |
| | $K_0$ | 0.3245 | 0.4811 | 0.5034 | 0.4779 |
| First order | $r^2$ | 0.9562 | 0.9648 | 0.9632 | 0.9809 |
| | $K_1$ | 0.0011 | 0.0017 | 0.0022 | 0.0023 |
| Hixon-crowell | $r^2$ | 0.9432 | 0.9488 | 0.9422 | 0.9606 |
| | $K_{HC}$ | 0.0072 | 0.0113 | 0.0133 | 0.0135 |
| Higuchi | $r^2$ | 0.9794 | 0.9817 | 0.9711 | 0.9803 |
| | $K_H$ | 4.4085 | 6.5597 | 6.9091 | 6.5298 |
| Korsmeyer-Peppas | $r^2$ | 0.9891 | 0.9827 | 0.9825 | 0.9926 |
| | $K_{KP}$ | 11.4128 | 6.2598 | 9.9872 | 14.2658 |

As shown in Table 4 and FIG. 2, lower PLGA ratios resulted in faster dissolution rates. The microspheres of Example 1 and Example 2 exhibited a drug release of approximately 77.7% to 81% at 120 hours, making them suitable for one week sustained-release.

As shown in Table 5, the dissolution pattern shows a sustained-release pattern and fits the Higuchi or Korsmeyer-Peppas kinetics.

Based on these analysis results, the sustained-release estrogen microspheres of the present invention are optimally prepared by mixing 400 to 600 parts by weight of a biodegradable polymer with 100 parts by weight of estrogen and stirring at a speed of 1600 rpm or more for at least 12 hours.

**Preparation Example 2: Determining Conditions for Desogestrel Microsphere preparation**

<Microsphere preparation>

Desogestrel (DSG) and PLGA 503H (Evonik Ltd. Germany) were added to 10 ml of dichloromethane (DCM) in the amounts shown in Table 6, mixed, and dissolved by sonication for 30 minutes to prepare an oil phase. The oil phase was dropped into 100 ml of polyvinyl alcohol solution (5% PVA in D.W) while stirring at the stirring speeds shown in Table 6, using a syringe pump at a rate of 2 ml/min to allow DCM to evaporate, for 12 hours, thus forming microspheres. The produced microspheres were precipitated using a centrifuge (2000 rpm for 10 minutes). The precipitated microspheres were filtered through a 0.2 $\mu$m PVDF membrane filter, washed twice with D.W, and dried at room temperature for 12 hours to to obtain the microspheres.

**Table 6**

| Component | Comparative example 4 | | Comparative example 5 | | Comparative example 6 | |
|---|---|---|---|---|---|---|
| | MD-3 (1:20) | | MD-4 (1:10) | | MD-5 (1:10) | |
| | mg | % | mg | % | mg | % |
| PLGA | 600 | 95.2 | 600 | 90.9 | 600 | 90.9 |
| DSG | 30 | 4.8 | 60 | 9.1 | 60 | 9.1 |
| DCM (ml) | 10 | - | 10 | - | 10 | - |
| Total (Solute) | 660 | 100.0 | 660 | 100.0 | 660 | 100.0 |
| rpm | 2000 | | 800 | | 1600 | |

<Yield, Loading, and Encapsulation Efficiency of Microspheres>

Using HPLC equipment (Agilent 1200 HPLC system), the drug encapsulated in the produced microspheres was analyzed, and the yield, loading, and encapsulation efficiency of the microspheres were calculated using the above formulas in Preparation Example 1 and shown in Table 7.

**Table 7**

| | Comparative example 4 | Comparative example 5 | Comparative example 6 |
|---|---|---|---|
| | MD-3 | MD-4 | MD-5 |
| Drug | DSG | DSG | DSG |
| Yield (%) | 73.3 | 79.0 | 82.7 |
| Loading (%) | 3.81±0.05 | 7.50±0.84 | 7.46±0.61 |
| Encapsulation efficiency(%) | 80.11±1.23 | 82.53±9.27 | 82.07±6.8 |

<Particle Shape and Size of Microspheres>

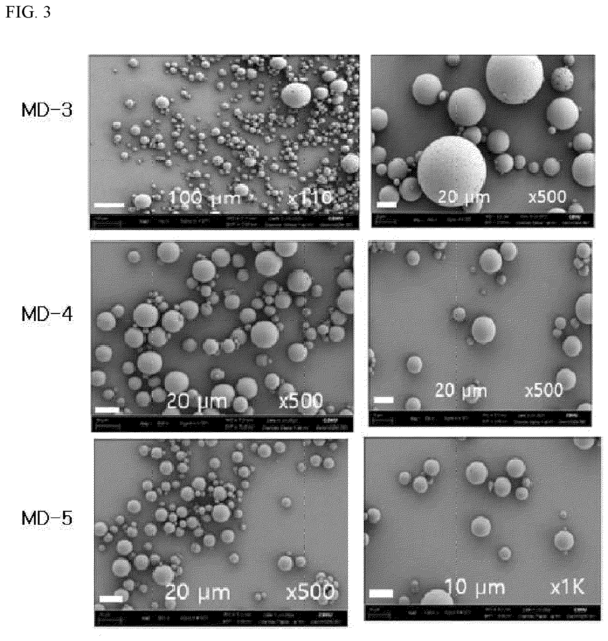

Particle shapes of the microspheres were observed using SEM equipment (Ultra Plus Carl Zeiss), and the results are shown in FIG. 3. All microspheres exhibited a spherical shape.

Additionally, particle sizes (Dv10, Dv50, Dv90) of the microspheres were measured using a Mastersizer 3000E equipment (wet measurement), and the results are shown in Table 8.

**Table 8**

| | | Dv10 ($\mu$m) | Dv50 ($\mu$m) | Dv90 ($\mu$m) | Span |
|---|---|---|---|---|---|
| Comparative example 4 | MD-3 | 14.00±0.10 | 27.90±0.08 | 55.00±1.42 | 1.47±0.04 |
| Comparative example 5 | MD-4 | 10.03±0.06 | 20.37±0.21 | 40.40±1.49 | 1.49±0.06 |
| Comparative example 6 | MD-5 | 3.71±0.00 | 8.20±0.03 | 12.60±0.01 | 1.08±0.01 |

Comparative Example 4 and Comparative Example 5 showed a particle size (Dv90) of approximately 55 $\mu$m, making them unsuitable for use in microneedles. It was confirmed that larger amounts of the biodegradable polymer increase the microsphere size excessively. Additionally, it can be confirmed that stirring at 1600 rpm or higher during the preparation of microspheres results in a Dv90 of around 10 $\mu$m, which is a desirable size for incorporation into microneedles.

<Dissolution Test - Sustained Release Test>

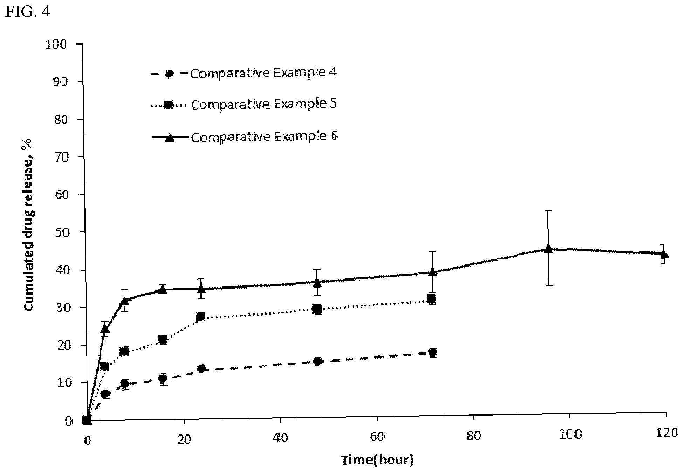

The sustained-release characteristics were analyzed by performing a dissolution test on the microspheres prepared as described above, following the same method as in Example 1. The test results are shown in FIG. 4 and Table 9.

**Table 9**

| Time (hr) | Comparative example 4 | Comparative example 5 | Comparative example 6 |
|---|---|---|---|
| | MD-3 | MD-4 | MD-5 |
| 0 | 0 | 0 | 0 |
| 4 | 6.80±0.93 | 14.0±0.54 | 25.1±0.46 |
| 8 | 9.38±1.48 | 17.9±0.57 | 28.3±0.27 |
| 16 | 10.5±1.54 | 20.9±0.95 | 31.2±0.25 |
| 24 | 12.8±0.25 | 26.8±0.61 | 32.4±0.15 |
| 48 | 14.7±0.28 | 28.6±1.33 | 40.6±0.44 |
| 72 | 16.7±1.37 | 30.6±1.08 | 37.7±1.37 |
| 96 | - | - | 42.2±0.54 |
| 120 | - | - | 44.1±1.57 |

As shown in Table 9 and FIG. 4, the microspheres of Comparative Example 4 to Comparative Example 6 showed drug release of less than approximately 44% at 120 hours, making them unsuitable for one week sustained-release, and requiring improvements in the stability of the drug (DSG).

**Preparation Example 3: Preparation of Desogestrel Microspheres with Different Stabilizers**

<Selection of Stabilizers>

To select suitable stabilizers for preparing desogestrel microspheres with enhanced stability, compositions containing stabilizers were prepared as shown in Table 10, and thin films were prepared by using the compositions for stability testing.

**Table 10**

| Content (mg) | Composition 1 | Composition 2 | Composition 3 | Composition 4 |
|---|---|---|---|---|
| DSG | 5 | 5 | 5 | 5 |
| PLGA | 50 | 50 | 50 | 50 |
| Hydroxypropyl-beta-cyclodextrin (HPβCD) | | 58 | - | - |
| Tocopherol | - | - | 36 | - |
| BHA (Butylated hydroxyanisole) | - | - | - | 36 |

Specifically, the ethanol solution in which the drug (DSG) and each stabilizer were dissolved was mixed with the DCM solution containing PLGA. After mixing, the mixture was dried using a nitrogen evaporator to form a thin film with a thickness of 50-100 μm.

Each thin film was subjected to stability analysis immediately after production and after storage for 8 weeks in a temperature and humidity-controlled chamber at 30°C and 65% relative humidity (intermediate accelerated conditions).

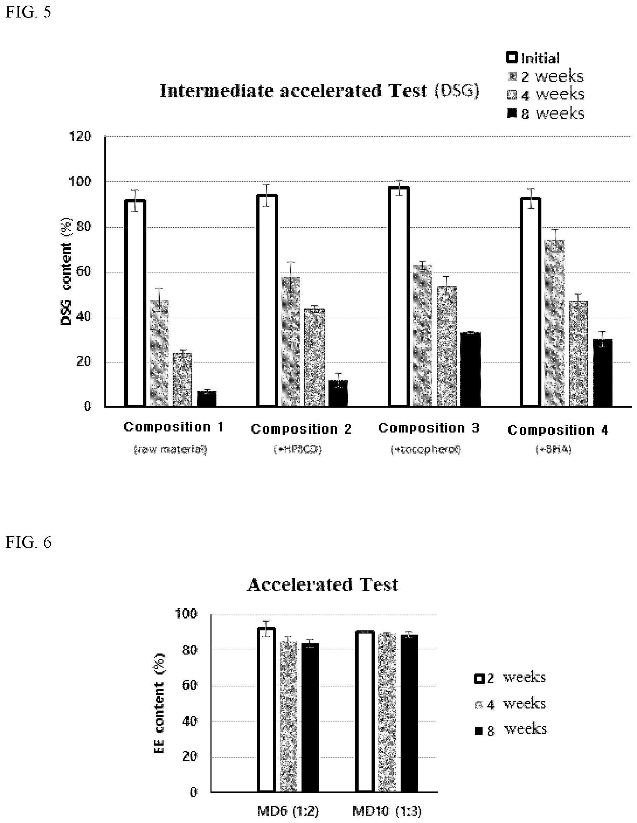

Specifically, for the stability analysis, each film was completely dissolved in 5 ml of a diluent solvent (a 1:1 mixture of phosphate buffer solution at pH 3.5 and 78% acetonitrile). The resulting solution was shaken to mix thoroughly and then subjected to quantitative analysis of DSG using HPLC under the conditions described in Table 11 below. The results are shown in Table 12 and FIG. 5.

**Table 11**

| Instrument | Thermo U3000 |
|---|---|
| Column | C8, 4.6 * 100 mm, 3.5 μm |
| Wavelength | 215 nm |
| Column temprerature | 45 °C |

(continued)

| Flow rate | 1.0 ml/min |
|---|---|
| Injection volume | 10 μl |
| Run time | 40 min |
| Mobile phase | A: Phosphate buffer solution (pH 3.5) |
| | B: 78% Acetonitrile |
| Gradient | 0-40 min: 56-64% B |

**Table 12**

| content (%) | Composition 1 | Composition 2 | Composition 3 | Composition 4 |
|---|---|---|---|---|
| Initial | 91.45±4.95 | 102.04±1.38 | 93.82±4.97 | 97.32±3.39 |
| After 2 weeks | 47.33±5.13 | 85.96±3.30 | 57.42±6.60 | 62.77±1.78 |
| After 4 weeks | 23.59±1.64 | 85.64±1.80 | 43.27±1.33 | 53.64±4.23 |
| After 8 weeks | 6.90±1.08 | 72.70±13.6 | 11.76±3.18 | 33.08±0.55 |

As shown in Table 12 and FIG. 5, it was confirmed that using hydroxypropyl-beta-cyclodextrin (HPβCD) as a stabilizer prevented DSG degradation and ensured stability after 8 weeks under intermediate accelerated conditions.

<Preparation of Microspheres with Stabilizer and Composition Ratio>

HPβCD was added to 2 ml of D.W in the amounts shown in Table 13 and dissolved by mixing and sonication for 30 minutes to produce an aqueous phase. Desogestrel (DSG) and PLGA 503H (Evonik Ltd. Germany) were added to 10 ml of dichloromethane (DCM) and dissolved by mixing and sonication for 30 minutes to produce an oil phase. The aqueous phase and the oil phase were mixed and homogenized at 12000 rpm for 10 seconds to prepare a mixed solution. While stirring 100 ml of polyvinyl alcohol solution (5% PVA in D.W) at 1600 rpm, the mixed solution was added dropwise to the polyvinyl alcohol solution at a rate of 2 ml/min using a syringe pump. Stirring was continued for 12 hours to allow the DCM to evaporate, resulting in the formation of microspheres. The microspheres were precipitated using a centrifuge (2000 rpm for 10 minutes). The precipitated microspheres were filtered using a 0.2 μm PVDF membrane filter, washed twice with D.W, and then dried at room temperature for 12 hours to complete the microsphere preparation.

**Table 13**

| | Component | MD-6 | MD-10 |
|---|---|---|---|
| | | mg | mg |
| Inner water phase | HPβCD (DSG:HPβCD molar ratio) | 705 (1:2) | 1058 (1:3) |
| | D.W (ml) | 2 | 2 |
| Inner oil phase | PLGA | 600 | 600 |
| | DSG | 75 | 75 |
| | Total (Solute) | 1380 | 1733 |

For the prepared microspheres, stability analysis was performed immediately after production and after 8 weeks of storage in a temperature and humidity-controlled chamber at 40°C and 75% relative humidity (accelerated conditions). The stability analysis involved quantifying DSG using the same HPLC method as described above. The results are shown in Table 14 and FIG. 6.

**Table 14**

| Components | | DSG (%) | | |
|---|---|---|---|---|
| Formulation | DSG:HPβCD (mole) | 2 weeks | 4 weeks | 8 weeks |
| MD-6 | 1:2 | 91.97±1.49 | 84.88±2.09 | 83.52±5.53 |
| MD-10 | 1:3 | 90.28±6.05 | 88.83±1.56 | 88.40±1.61 |

As shown in Table 14 and FIG. 6, the microspheres prepared by adding hydroxypropyl-beta-cyclodextrin (HPβCD) as a stabilizer to desogestrel (DSG) at a molar ratio of 1:2 to 1:3 demonstrated maintained stability, as evidenced by the prevention of DSG degradation even after 8 weeks of storage under accelerated conditions.

**Preparation Example 4: Preparation of Sustained-Release Desogestrel Microspheres**

<Microsphere Preparation>

HPβCD 705 mg was added to 2 ml of D.W and dissolved by mixing and sonication for 30 minutes to prepare an aqueous phase. DSG and PLGA 503H (50:50 of lactic: glycolic), 753H (75:25), 203H (100:0), 503 (50:50) (Evonik Ltd. Germany) were each added in the amounts shown in Table 15 to 10 ml of dichloromethane (DCM), and mixed and dissolved by sonication for 30 minutes to prepare an oil phase. The aqueous phase and the oil phase were mixed and homogenized at 12,000 rpm for 10 seconds to prepare a mixed solution. While stirring 100 ml of polyvinyl alcohol solution (5% PVA in D.W) at 1600 rpm, the mixed solution was added dropwise to the polyvinyl alcohol solution at a rate of 2 ml/min using a syringe pump. Stirring was continued for 12 hours to allow the DCM to evaporate, resulting in the formation of microspheres. Then the microspheres were precipitated using a centrifuge (2000 rpm for 10 minutes). The precipitated microspheres were filtered using a 0.2 μm PVDF membrane filter, washed twice with D.W, and dried at room temperature for 12 hours to complete the microsphere preparation.

**Table 15**

| | Component | Comparative example 7 | Example 3 | Comparative example 8 | Comparative example 9 | |
|---|---|---|---|---|---|---|
| | | MD-6 | MD-18 | MD-19 | MD-20 | |
| | | mg | mg | mg | mg | |
| Inner water phase | HPβCD | 705 | 705 | 705 | 705 | |
| | D.W (ml) | 2 | 2 | 3 | 3 | |
| Inner oil phase | PLGA (lactic: glycolic) 503H (50:50) | 600 | - | - | - | |
| | 753H (75:25) | - | 600 | - | - | |
| | 203H (100:0) | - | - | 600 | - | |
| | 503 (50:50) | - | - | - | 600 | |
| | DSG | 75 | 75 | 75 | 75 | |
| | DCM (ml) | 10 | 10 | 10 | 10 | |
| | Total (Solute) | 1380 | 1380 | 1380 | 1380 | |

<Yield, Loading, and Encapsulation Efficiency of Microspheres>

Using HPLC equipment (Agilent 1200 HPLC system), the drug encapsulated in the produced microspheres was analyzed, and the yield, loading, and encapsulation efficiency of the microspheres were calculated using the above formulas in Preparation Example 1 and shown in Table 16.

**Table 16**

| | Comparative example 7 | Example 3 | Comparative example 8 | Comparative example 9 |
|---|---|---|---|---|
| | MD-6 | MD-18 | MD-19 | MD-20 |
| Drug | DSG | DSG | DSG | DSG |
| Yield (%) | 38.4 | 38.0 | 37.9 | 34.4 |
| Loading (%) | 7.52±0.09 | 9.08±0.15 | 9.63±0.02 | 6.57±0.02 |
| Encapsulation efficiency(%) | - | 78.76±0.35 | 46.02±2.15 | 81.11±0.39 |

The yield was similar for different PLGA grades, and PLGA formulations with higher lactic/glycolic ratios (MD-18 or MD-19) showed a 1-1.5% increase in loading.

<Particle Shape and Size of Microspheres>

Particle shapes of the microspheres were observed using SEM equipment (Ultra Plus Carl Zeiss), and the results are shown in FIG. 7. Pores were observed on the surface of MD-18 and MD-19 microspheres. This is likely due to the increased hydrophobicity of the outer phase, causing the hydrophilic solvent of the inner phase to diffuse out more quickly.

Additionally, particle sizes (Dv10, Dv50, Dv90) of the microspheres were measured using a Mastersizer 3000E equipment (wet measurement), and the results are shown in Table 17.

**Table 17**

| | | Dv10 (μm) | Dv50 (μm) | Dv90 (μm) | Span |
|---|---|---|---|---|---|
| Comparative example 7 | MD-6 | 2.53±0.01 | 7.98±0.03 | 23.47±1.08 | 2.62±0.12 |
| Example 3 | MD-18 | 4.056±0.02 | 8.13±0.03 | 18.63±0.49 | 1.79±0.05 |
| Comparative example 8 | MD-19 | 3.76±0.00 | 7.12±0.01 | 12.90±0.20 | 1.28±0.02 |
| Comparative example 9 | MD-20 | 3.546±0.01 | 7.05±0.00 | 18.07±0.06 | 2.05±0.00 |

For the microspheres in comparison example 7, the particle size (Dv90) was approximately 23 μm, which was too large for use(incorporation) in micronization.

<Dissolution Test - Sustained Release Test>

The sustained-release characteristics were analyzed by performing a dissolution test on the microspheres prepared as described above, following the same method as in Example 1. The test results are shown in FIG. 8 and Table 18.

**Table 18**

| Time (hr) | Comparative example 7 | Example 3 | Comparative example 8 | Comparative example 9 |
|---|---|---|---|---|
| | MD-6 | MD-18 | MD-19 | MD-20 |
| | DSG | DSG | DSG | DSG |
| 0 | 0 | 0 | 0 | 0 |
| 4 | 20.41±0.16 | 23.58±2.02 | 13.81±0.50 | 21.12±1.74 |
| 8 | 24.61±0.14 | 30.88±0.50 | 17.78±0.36 | 26.57±2.74 |
| 16 | 30.21±0.20 | 41.33±1.81 | 24.20±0.78 | 32.84±1.49 |
| 24 | 34.60±0.44 | 48.14±0.53 | 29.02±0.51 | 40.76±0.22 |
| 48 | 43.70±0.12 | - | 35.99±1.44 | 49.68±2.43 |
| 72 | 52.54±0.31 | 67.98±1.11 | 46.37±1.34 | 58.52±0.34 |
| 96 | 59.19±0.57 | - | 47.99±0.53 | 59.94±0.61 |

(continued)

| Time (hr) | Comparative example 7 | Example 3 | Comparative example 8 | Comparative example 9 |
|---|---|---|---|---|
| | MD-6 | MD-18 | MD-19 | MD-20 |
| | DSG | DSG | DSG | DSG |
| 120 | 59.93±1.53 | 75.08±1.28 | 55.07±1.42 | 65.98±0.66 |

As shown in Table 18 and FIG. 8, the microspheres in Example 3 exhibited approximately 75% drug release at 120 hours, indicating suitability for one week sustained-release.

Due to the influence of pores on the surface of the microspheres, microspheres of Example 3 (MD-18) exhibited a rapid release profile after 4 hours, while the microspheres of comparative example 8 (MD-19) showed a delayed elution profile due to an increased lactic/glycolic ratio (increased hydrophobicity) despite the presence of pores.

As a result of the above analysis, the sustained-release progesterone microspheres of the present invention are preferable to comprise 700 to 900 parts by weight of a biodegradable polymer for every 100 parts by weight of progesterone. In addition hydroxypropyl-beta-cyclodextrin is desirable to be included in the microspheres at a molar ratio of progesterone to hydroxypropyl-beta-cyclodextrin ranging from 1:2 to 1:3. It is desirable that the biodegradable polymer has a glycolic : lactic ratio of 1 : 2 to 4, and the stirring during preparation of microspheres is carried out at a speed of at least 1600 rpm for at least 12 hours.

**Preparation Example 5: Preparation of contraceptive microneedles**

< Preparation of a Solution for Microneedle Preparation>

Ethinylestradiol microspheres of Example 2 and desogestrel microspheres of Example 3 (FIG. 9) were used to prepare a solution for microneedle preparation with the formulations shown in Table 19:

**Table 19**

| Content (% by weight) | Example 4 | Example 5 | Example 6 |
|---|---|---|---|
| Ethinylestradiol microspheres (Example 2, ME-6) | -. | 1.43 | 1.43 |
| Desogestrel microspheres (Example 3, MD-18) | 5.07 | -. | 5.07 |
| HA | 25.50 | 26.48 | 25.12 |
| Xylitol | 5.67 | 5.88 | 5.58 |
| D.W | 63.76 | 66.21 | 62.80 |
| Total | 100.00 | 100.00 | 100.00 |

Specifically, the microspheres (119.23 mg of MD-18 for Example 4, 32.40 mg of ME-6 for Example 5, 121.13 mg of MD-18 and 34.17 mg of ME-6 for Example 6) were added to the reactor, followed by 1500 mg of purified water. And the mixture in the reactor was then subjected to 3 minutes of vortexing and 2 minutes of sonication to ensure there were no clumped(agglomerated) microspheres. 133.33 mg of xylitol was added to the reactor and subjected to 3 minutes of vortexing and 2 minutes of sonication until completely dissolved. 600 mg of hyaluronic acid (HA; sodium hyaluronate) was added to the reactor and subjected to 5 minutes of vortexing and 2 minutes of sonication until completely dissolved, and then was deaerated under reduced pressure to obtain a solution for preparation of microneedles.

<Preparation of microneedles >

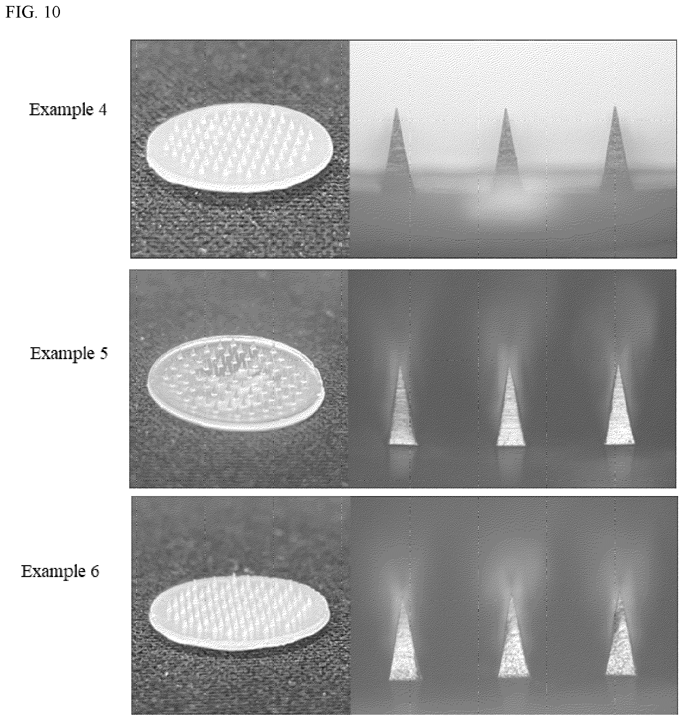

0.2 g of the solution prepared above was loaded into a PMDS negative mold with a circular patch-shape. After loading, the molds were placed in a desiccator and subjected to reduced pressure to ensure the solution filled the negative mold. After using compressed air to remove any bubbles, they were dried at room temperature for 16 hours. The prepared microneedles were then separated from the molds. A photograph of the prepared microneedles and a photograph of the needle parts are shown in FIG. 10.

As needed, if necessary, the microneedles can have a colloid band or similar attached to the back to enhance usability in a patch form.

As shown in FIG. 10, it is confirmed that the microneedles of Examples 4 to 6 are well formed and the microspheres are enclosed in their needle parts.

**Experimental Example 1: Analysis of strength and drug content of microneedles**

<Strength of Microneedles>

For each of the microneedles of Examples 4 to 6 prepared in Preparation Example 5, compressive strength was measured using a universal material testing machine (Instron 34sc-05) at a test speed of 0.1 mm/s and a test end condition of 100 N. For data analysis, for each prepared microneedle, the force value corresponding to the compressive displacement at a point 1/3 of the total length of the needle parts was taken, and the measured force value/number of needles formula was used to calculate the force value of one microneedle. The results are shown in Table 20.

**Table 20**

| Metrics | Example 4 | Example 5 | Example 6 |
|---|---|---|---|
| Needle compressive strength (N/needle number) | 0.14 | 0.12 | 0.21 |

As shown in Table 20, the microneedles of Examples 4-6 had sufficient strength to penetrate the skin.

<Analysis of Drug Content of Microneedles >

For each of the microneedles of Examples 4 to 6 prepared in Preparation Example 5, the drug content was analyzed using HPLC (Agilient 1260 Infinity II Prime LC). As a sample, each microneedle was dissolved in 1 ml of 50% ACN aqueous solution. The analysis was performed under conditions as shown in Table 21 (desogestrel) and Table 22 (ethinylestradiol) below, and the results are shown in Table 23.

**Table 21**

| | |
|---|---|
| Column | YMC-Pack ODS-AQ / 250*4.6mm / 5um |
| Mobile Phase | acn:dw = 73:27 |
| Flow rate | 1.0 mL/min |
| Detector | DAD (205 $\mu$m) |
| Run Time | 35 min |
| Column Temp. | 50 |
| Injection Volume | 15 $\mu$L |
| Sample solution | 50% ACN aq. |

**Table 22**

| | |
|---|---|
| Column | Shiseido CAPCELL PAK C18 MG / 150*4.6mm / Sum |
| Mobile Phase | acn:dw = 1:1 |
| Flow rate | 1.0 mL/min |
| Detector | DAD (280 $\mu$m) |
| Run Time | 15min |
| Column Temp. | 25 |
| Injection Volume | 25 $\mu$L |
| Sample solution | 50% ACN aq. |

**Table 23**

| | Example 4 | Example 5 | Example 6 |
|---|---|---|---|
| Desogestrel Content (mg) | 0.513 | -. | 0.697 |

(continued)

|  | Example 4 | Example 5 | Example 6 |
|---|---|---|---|
| Ethinylestradiol Content (mg) | -. | 0.070 | 0.053 |

As shown in Table 23, it is confirmed that the microspheres are adequately incorporated into the microneedles.

## Claims

1.  A microsphere composition for contraceptive microneedles, comprising:

    one selected from the group consisting of sustained-release estrogen microspheres, sustained-release progesterone microspheres, and mixtures thereof,
    wherein said sustained-release estrogen microspheres comprise 400 to 600 parts by weight of a biodegradable polymer per 100 parts by weight of estrogen, and wherein said biodegradable polymer is any one selected from the group consisting of poly(lactide-co-glycolide) (PLGA), polylactide (PLA), polyglycolide (PGA), poly(lactide-co-glycolide) glucose, and mixtures thereof, and wherein stirring during preparation of the microspheres is performed at a speed of 1600 rpm or higher for at least 12 hours, and wherein the particle size (Dv90) of the microspheres is 20 μm or less, and 70-80% by weight of estrogen from the microspheres is continuously released within 120 hours;
    wherein said sustained-release progesterone microspheres comprise 700 to 900 parts by weight of a biodegradable polymer per 100 parts by weight of progesterone and hydroxypropyl-beta-cyclodextrin (HPβCD) at a molar ratio of progesterone to hydroxypropyl-beta-cyclodextrin of 1:2 to 1:3, and wherein said biodegradable polymer is any one selected from the group consisting of poly(lactide-co-glycolide) (PLGA) with a glycolic:lactic ratio of 1:2 to 4, poly(lactide-co-glycolide) glucose, and mixtures thereof, and wherein the particle size (Dv90) of the microspheres is 20 μm or less, and 70-80% by weight of the progesterone from the microspheres is continuously released within 120 hours.

2.  The microsphere composition for contraceptive microneedles according to claim 1, wherein said estrogen is ethinylestradiol, and said progesterone is any one selected from the group consisting of desogestrel, gestodene, dienogest, levonorgestrel, norgestimate, norethisterone, dro-spirenone, trimegestone, and dydrogesterone.

3.  The microsphere composition for contraceptive microneedles according to claim 1, wherein the sustained-release estrogen microspheres comprise 400 parts by weight of a biodegradable polymer per 100 parts by weight of estrogen, and wherein said biodegradable polymer is poly(lactide-co-glycolide) (PLGA).

4.  The microsphere composition for contraceptive microneedles according to claim 1, wherein the sustained-release progesterone microspheres comprise 700 parts by weight of a biodegradable polymer per 100 parts by weight of progesterone and hydroxypropyl-beta-cyclodextrin (HPβCD) at a molar ratio of progesterone to hydroxypropyl-beta-cyclodextrin of 1:2.

5.  The microsphere composition for contraceptive microneedles according to claim 1, wherein the biodegradable polymer of the sustained-release progesterone microspheres is poly(lactide-co-glycolide) (PLGA) with a glycolic:lactic ratio of 1:3.

6.  The microsphere composition for contraceptive microneedles according to claim 1, wherein the sustained-release progesterone microspheres have pores formed on their surface.

7.  The microsphere composition for contraceptive microneedles according to claim 1, wherein the composition comprises only sustained-release estrogen microspheres.

8.  The microsphere composition for contraceptive microneedles according to claim 1, wherein the composition comprises only sustained-release progesterone microspheres.

9.  The microsphere composition for contraceptive microneedles according to claim 1, wherein the composition comprises both sustained-release estrogen microspheres and sustained-release progesterone microspheres.

10. Contraceptive microneedles prepared from the composition according to any one of claims 1 to 9, wherein the microneedles comprise needle parts protruding in one direction and a matrix layer supporting the needle parts.

11. The contraceptive microneedles according to claim 10, wherein the needle parts are separable from the matrix layer when inserted into the skin.

12. The contraceptive microneedles according to claim 10, wherein the microneedles are prepared using a mold.

13. A contraceptive microneedle patch comprising the microneedles according to claim 10.

14. The contraceptive microneedle patch according to claim 13, wherein the contraceptive microneedle patch is used once a week.

FIG. 1

FIG. 2

FIG. 3

FIG. 4

FIG. 5

Intermediate accelerated Test (DSG)

□ Initial
2 weeks
4 weeks
■ 8 weeks

FIG. 6

Accelerated Test

□ 2 weeks
4 weeks
■ 8 weeks

FIG. 7

MD-6

MD-18

MD-19

MD-20

FIG. 8

FIG. 9

ME-6                    MD-18

FIG. 10

Example 4

Example 5

Example 6

## EUROPEAN SEARCH REPORT

**Application Number**

EP 24 18 5615

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 2023 0104511 A (SMALLLAB CO LTD [KR]) 10 July 2023 (2023-07-10) | 1-3,5-14 | INV. A61K9/00 |
| A | * paragraphs [0001], [0005], [0005], [0007] * | 4 | A61K9/50 A61K45/06 |
| | * paragraphs [0010] - [0012], [0015], [0016] * | | A61M37/00 A61K31/567 |
| | * paragraphs [0020], [0026], [0027] * | | |
| | * paragraphs [0041], [0047], [0048] * | | |
| | * paragraphs [0057], [0076] * | | |
| | * claim 1 * | | |
| | ----- | | |
| A | GUO YUTING ET AL: "Injectable Sustained-Release Depots of PLGA Microspheres for Insoluble Drugs Prepared by hot-Melt Extrusion", ENVIRONMENTAL SCIENCE AND POLLUTION RESEARCH, SPRINGER BERLIN HEIDELBERG, BERLIN/HEIDELBERG, vol. 34, no. 10, 24 July 2017 (2017-07-24), pages 2211-2222, XP036310069, ISSN: 0724-8741, DOI: 10.1007/S11095-017-2228-X [retrieved on 2017-07-24] * the whole document * | 1-14 | |
| | ----- | | |

**TECHNICAL FIELDS SEARCHED (IPC)**

A61K
A61M

The present search report has been drawn up for all claims

| Place of search | Date of completion of the search | Examiner |
|---|---|---|
| The Hague | 26 November 2024 | Weiss, Marie-France |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 5615

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-2024

| Patent document cited in search report | Publication date | Patent family member(s) | Publication date |
|---|---|---|---|
| KR 20230104511 A | 10-07-2023 | NONE | |

**EP 4 578 442 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020070087141

- KR 100758757